# DEMANDE DE BREVET EUROPEEN

(11) **EP 1 230 941 A1**
(43) Date de publication de la demande: **14.08.2002**
(21) Numéro de dépôt: 01810137.8
(22) Date de dépôt: 09.02.2001
(51) Int. Cl.: A61M 5/158

(54) **Aiguille à deux lumières**

(71) Demandeur: Rochat, Jean-Denis, 1272 Genolier (CH)
(72) Inventeur: Rochat, Jean-Denis, 1272 Genolier (CH)
(74) Mandataire: Savoye, Jean-Paul

(57) **Abrégé**

Cette aiguille à deux lumières comporte une extrémité d'évacuation située à l'extrémité distale perforante (7a) d'un tube rigide (7) et une extrémité d'admission (8) située en retrait et s'ouvre latéralement à la lumière de prélèvement (4). Elle comporte un embout (2) dont l'extrémité proximale (2c) dont la partie distale (2a) est de section circulaire, se termine entre l'extrémité d'évacuation (7a) de la lumière de retour (3) et l'extrémité d'admission (8) de la lumière de prélèvement (4) et présente, disposés longitudinalement côte à côte, d'une part un siège de fixation (5) dudit tube rigide (7) et d'autre part ladite lumière de prélèvement (4), l'extrémité distale (2a) dudit embout formant ainsi une brusque augmentation de section de ladite aiguille située dans le prolongement de ladite lumière de prélèvement (4).

## Description

La présente invention se rapporte à une aiguille à deux lumières pour prélever et simultanément retourner du sang dans une veine d'un être vivant, dans laquelle l'extrémité d'évacuation de la lumière de retour du sang est située à l'extrémité distale perforante d'un tube rigide, tandis que l'extrémité d'admission de la lumière de prélèvement est située longitudinalement en retrait de ladite extrémité d'évacuation et s'ouvre latéralement à cette lumière de prélèvement, les extrémités proximales desdites lumières comportant chacune des moyens de raccordement à un conduit.

Il existe un très grand nombre de cathéters à deux lumières destinés à permettre de retirer et simultanément de retourner du sang à un être vivant. Ce type de cathéters est utilisé en particulier pour la dialyse et doit permettre d'assurer des débits élevés de l'ordre de 200 ml jusqu'à 300 ml par minute. Les diamètres des lumières de tels cathéters sont évidemment en rapport avec le débit recherché.

On a proposé dans le US 4 842 582 un cathéter de ce type comprenant une aiguille à deux lumières présentant une extrémité distale biseauté et cylindrique formant le conduit de retour du sang, suivie d'une portion conique. Un conduit interne de section semi-circulaire formant une des deux lumières vient s'ajuster dans la parti distale cylindrique. Ce conduit interne de section constante suit la paroi de la portion conique de l'aiguille sur une moitié environ de sa section, de sorte qu'une seconde lumière se forme progressivement, au fur et à mesure que la section de l'aiguille augmente. Des ouvertures sont ménagées dans la paroi délimitant l'autre moitié de la section de la portion conique de l'aiguille et servant d'ouvertures d'admission pour le prélèvement du sang dans cette seconde lumière.

L'utilisation de ce cathéter est prévue avec un garrot disposé entre les ouvertures d'évacuation, respectivement d'admission distantes longitudinalement des deux lumières, séparant ainsi la veine entre sa partie amont, qui est en communication avec l'ouverture d'admission et sa partie aval, qui est en communication avec l'ouverture d'évacuation. Ce garrot permet d'augmenter le débit de sang prélevé.

La réalisation d'une telle aiguille est relativement difficile. Il faut former la partie conique à partir d'un tube cylindrique, il faut mettre en forme et ajuster le tube interne dans le tube externe. Ensuite l'utilisation n'est pas simple étant donné que la distance longitudinale qui sépare les ouvertures d'admission et d'évacuation est forcément courte, la largeur du garrot doit être très faible, ce qui peut rendre sa pose douloureuse à la longue et son positionnement doit être très précis sous peine de fermer une des ouvertures ou de ne pas se trouver entre elles.

En outre, un tel cathéter est essentiellement prévu pour la dialyse. Si il constitue sans doute une amélioration dans ce contexte, il faut constater que cette solution ne s'adapte pas aux cas où il s'agit de prélever du sang pour en extraire une fraction, plaquettes ou plasma par exemple et de retourner le reste au donneur en travaillant en continu, avec des débits typiquement inférieurs à 100 ml/min.

Dans ce genre de prélèvement, on utilise essentiellement des systèmes discontinus dans lesquels on prélève et alternativement on retourne les parties de sang restantes au donneur, pour ne pas avoir à percer la veine en deux endroits. De ce fait, le temps nécessaire au prélèvement dure deux fois plus longtemps que si l'on travaillait en continu.

On a proposé de remédier à cet inconvénient en stockant le sang à retourner au donneur et en le réinjectant à grand débit. Or, pour éviter que le sang ne risque de former des caillots, on le mélange avec un produit anti-coagulant, de sorte qu'en réinjectant ce sang avec l'anti-coagulant dans la circulation sanguine du donneur à grand débit, il y a un risque qu'une réaction se produise chez celui-ci en raison de la présence de l'agent anti-coagulant mal supporté à haute dose par l'organisme. C'est la raison pour laquelle les prescriptions légales de certains pays ne prennent pas en compte le débit moyen du sang retourné au donneur, mais le débit maximum instantané en limitant ce dernier à une valeur relativement basse, compatible avec la capacité d'absorption de l'agent anti-coagulant par l'organisme. De ce fait, il n'est alors pas possible de gagner du temps en accélérant le débit de retour.

Le but de la présente invention est de remédier au moins en partie aux inconvénients sus-mentionnés.

A cet effet, cette invention a tout d'abord pour objet une aiguille à deux lumières pour prélever et simultanément retourner du sang dans une veine d'un être vivant, selon la revendication 1. Elle a ensuite pour objet un procédé de fabrication d'une aiguille à deux lumières selon lequel l'embout selon les revendications 1 et 8 est surmoulé sur le tube rigide.

L'aiguille à deux lumières selon la présente invention est bien adaptée aux opérations de séparation de composants du sang dans lesquelles le reste du sang est retourné au donneur. Cette aiguille permet d'assurer en continu des débits compris entre 40 et 100 ml/min pour prélever le sang à traiter et de retourner le reste du sang avec un débit du même ordre de grandeur, diminué du volume de la partie prélevée. Au cas où le volume de la partie prélevée est important, ce volume peut être compensé en partie ou en totalité par l'adjonction d'un volume correspondant d'eau physiologique.

Cette aiguille ne nécessite pas de garrot. En effet, la brusque augmentation de section de cette aiguille située dans le prolongement de sa lumière de prélèvement formée à l'extrémité distale de son embout, permet d'obtenir un effet voisin de celui du garrot en formant une occlusion au moins partielle de la veine entre l'ouverture d'admission de la lumière de prélèvement et l'ouverture de sortie de la lumière de retour. Cette occlusion, même partielle, est suffisante pour assurer un débit de prélèvement du sang de l'ordre de 40 à 100 ml/min. Etant donné que ce débit est continu, aussi bien en prélèvement qu'en retour au donneur, il correspond en pratique à un débit moyen sensiblement plus élevé, si on le compare à celui des systèmes existants, où on alterne le prélèvement et le retour des parties restantes du sang, en faisant passer le sang prélevé et les parties de sang retourné par une aiguille à une seule lumière.

Un débit pratiquement constant du sang permet également de faire constamment travailler l'appareil de séparation du sang à son débit nominal sans stockage de sang et dans des conditions optimales. La section de la partie perforante de l'aiguille qui constitue aussi la lumière de retour n'a pas besoin d'être surdimensionnée pour faire passer temporairement un débit plus élevé. Or, plus la section de l'aiguille est réduite moins le traumatisme est grand pour le donneur.

Enfin, le coût de fabrication d'une telle aiguille est relativement bas et même si cette aiguille est plus chère qu'une aiguille à simple lumière, cette augmentation de prix reste compatible avec les avantages qui en découlent aussi bien du point de vue du temps gagné, de la diminution du traumatisme pour le donneur, de la simplicité d'utilisation, que des conditions de travail optimales dans lesquelles l'installation de séparation peut travailler.

Le dessin annexé illustre, schématiquement et à titre d'exemple, une forme d'exécution de l'aiguille à deux lumières objet de cette invention.
La figure 1 est une vue en coupe longitudinale de cette forme d'exécution, illustrant l'aiguille mise en place dans une veine du bras ;
la figure 2 est une vue en coupe agrandie de l'aiguille selon la ligne II-II de la figure 1.

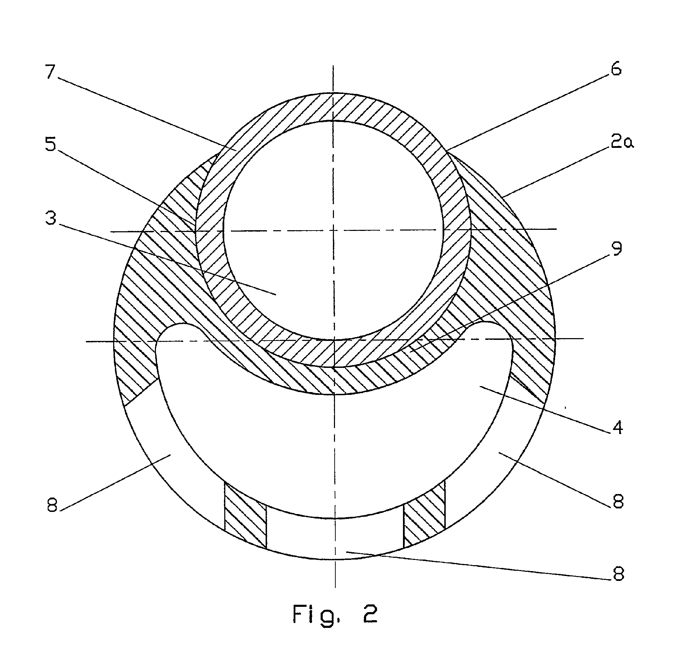

L'aiguille à deux lumières 1 comporte un embout 2, de préférence en matière plastique dans lequel sont ménagées deux lumières 3, 4. Cet embout 2 comporte une partie distale cylindrique 2a, une partie médiane conique 2b et une partie proximale de section constante 2c. La partie distale cylindrique 2a comporte, comme on le voit sur la figure 2, une surface de section circulaire 5 décentrée par rapport à l'axe longitudinal de l'embout 2. Le diamètre de la surface de section circulaire 5 décentrée est plus grand que le rayon de la partie distale cylindrique 2a. Cette surface 5 est disposée de manière d'une part, à s'ouvrir latéralement à l'extérieur de la partie distale 2a en formant ainsi une ouverture longitudinale 6 dont l'angle par rapport à l'axe central de la surface cylindrique 5 est inférieur à 180°, d'autre part à s'étendre au-delà de l'axe central de la partie distale cylindrique 2a de l'embout 2.

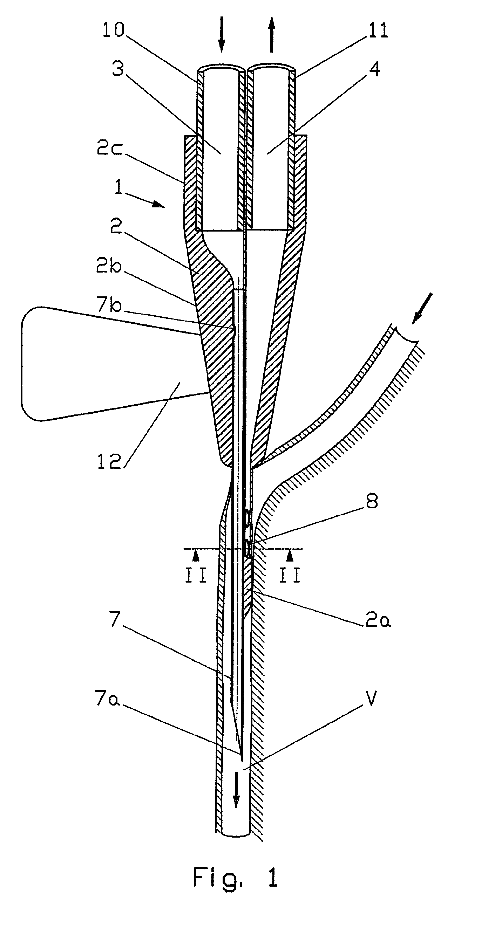

Cette surface cylindrique 5 pénètre ensuite dans la partie médiane conique 2b dans laquelle l'ouverture longitudinale 6 disparaît en raison de l'augmentation de section de cette partie médiane et forme une lumière. Suite à cette partie à surface cylindrique 5 la section de la lumière augmente pour rejoindre la partie proximale 3 de cette lumière. Cette surface cylindrique 5 constitue un siège de fixation d'un tube rigide 7 réalisé de préférence en acier inoxydable. Comme on le voit sur la figure 1, ce tube rigide 7 s'étend sensiblement au-devant de l'extrémité distale de l'embout 2 et son extrémité se termine par un biseau 7a formant ainsi une partie coupante, apte à traverser les tissus pour pénétrer dans une veine V, correspondant ici à une veine d'un bras. De préférence, l'extrémité proximale de ce tube 7 présente une ouverture latérale 7b pour permettre à la matière plastique de l'embout 2 d'ancrer le tube 7.

Comme illustré en particulier sur la figure 1, la seconde lumière 4 se termine à une certaine distance de l'extrémité distale de l'embout 2. Au moins une ouverture 8, de préférence plusieurs ouvertures, dans cet exemple deux séries espacées longitudinalement de trois ouvertures réparties angulairement, traversent la paroi latérale séparant la lumière 4 de l'extérieur de l'embout 2 à proximité de l'extrémité distale de cette lumière 4.

On peut constater sur la figure 2 que la section transversale de la portion de la lumière 4 située dans la partie distale cylindrique 2a de l'embout 2 est délimitée essentiellement par deux arcs de cercles interne et externe. Le centre de l'arc de cercle interne coïncide avec l'axe central de la surface cylindrique 5, tandis que le centre de l'arc de cercle externe coïncide avec l'axe central de l'embout 2, donnant à la section de cette portion de lumière 4 une forme de croissant séparé du tube 7 par une cloison arquée 9, concentrique à ce tube 7. Grâce à cette forme de croissant entourant partiellement le tube 7, lui-même décentré par rapport à la portion distale de l'embout, les sections internes de ce tube 7 et de la partie distale de la lumière 4 peuvent être choisies sensiblement égales, alors que le diamètre de cette portion distale 2a est cependant sensiblement inférieur à ce qu'il serait si l'on disposait deux tubes de sections équivalentes côte à côte.

Comme on le voit sur la figure 1, selon une autre particularité découlant de cette disposition décentrée du tube 7, l'extrémité distale de l'embout 2 forme une surépaisseur du côté de ce tube 7 où se trouvent les ouvertures d'admission 8 de la lumière 4. Comme conséquence, on peut constater qu'à l'endroit de cette surépaisseur, l'aiguille à deux lumières 1 occupe presque toute la section de la veine V. Ainsi, cette surépaisseur remplace le garrot en favorisant l'écoulement du sang dans la lumière de prélèvement 4. Elle empêche également que la portion du sang retourné au donneur par la lumière 3 et sortant par l'extrémité distale du tube 7 ne puisse venir dans la lumière de prélèvement 4. Bien entendu, comme illustré par la figure 1, il est nécessaire que l'aiguille à deux lumières 1 soit mise en place dans la veine V dans le sens d'écoulement du sang dans cette veine, afin que le sang qui s'y écoule rencontre tout d'abord les ouvertures d'admission 8 de la lumière de prélèvement 4.

Comme illustré par la figure 1, les sections des extrémités proximales respectives des deux lumières 3, 4 sont conformées pour recevoir respectivement deux conduits souples 10, 11 pour ramener les parties non prélevées du sang au donneur, respectivement conduire le sang à une machine de traitement, notamment une centrifugeuse pour la séparation des composants du sang à prélever.

Avantageusement, l'aiguille à deux lumières 1 qui vient d'être décrite est réalisée par surmoulage de l'embout 2 en matière plastique sur le tube 7 en acier inoxydable. Comme on peut le constater, sur la figure 1, les sections croissantes des lumières 3, 4 de leurs extrémités distales à leurs extrémités proximales permettent de retirer par ces dernières extrémités les inserts de moulage (non représentés) qui auront servis à former ces lumières lors de l'opération de surmoulage.

A titre d'exemple, l'aiguille à deux lumières peut avoir les dimensions suivantes : le tube 7 a une longueur de 31,7 mm, un diamètre externe de 1 mm et un diamètre interne de 0,8 mm, correspondant à une section de 0,5 mm². Ce tube fait saillie d'environ 10 mm à l'extrémité distale de l'embout 2. La partie distale cylindrique 2a de cet embout 2 présente un diamètre de 1,6 mm et la section de la lumière 4 dans cette même partie distale 2a est de 0,45 mm².

Une veine V typique du bras dans laquelle l'aiguille à deux lumières 1 peut être implantée peut avoir un débit de 100 ml/min. Avec l'aiguille à deux lumières selon l'exemple susmentionné on peut obtenir un débit de sang dans la lumière de prélèvement 4 de l'ordre de 60 ml/min, de sorte que 40 ml/min s'écouleront dans cette veine en aval des ouvertures d'admission 8 de la lumière de prélèvement 4. Le débit de sang retourné au donneur par la lumière 3 après prélèvement des composants retirés du sang peut typiquement être de l'ordre de 45 ml/min, de sorte que le débit total dans la veine V en aval de l'aiguille à deux lumières 1 est de 85 ml/min.

Etant donné qu'il est préférable que les deux lumières 3, 4 de l'aiguille 1 occupent les positions relatives illustrées par la figure 1, il est avantageux d'utiliser un papillon de fixation 12 solidaire de l'aiguille 1 pour la fixer au bras du donneur afin de conserver ces positions relatives des lumières 3, 4 au cours du processus de prélèvement.

## Revendications

1. Aiguille à deux lumières pour prélever et simultanément retourner du sang dans une veine d'un être vivant, dans laquelle l'extrémité d'évacuation de la lumière de retour du sang (3) est située à l'extrémité distale perforante (7a) d'un tube rigide (7), tandis que l'extrémité d'admission (8) de la lumière de prélèvement (4) est située longitudinalement en retrait de ladite extrémité d'évacuation et s'ouvre latéralement à cette lumière de prélèvement (4), les extrémités proximales desdites lumières (3, 4) comportant chacune des moyens de raccordement à un conduit (10, 11), **caractérisée en ce qu'**elle comporte un embout (2) dont l'extrémité proximale (2c) présente lesdits moyens de raccordement desdites lumières (3, 4) auxdits conduits (10, 11) et dont la partie distale (2a) est de section circulaire, se termine entre l'extrémité d'évacuation (7a) de ladite lumière de retour (3) et l'extrémité d'admission (8) de ladite lumière de prélèvement (4) et présente, disposés longitudinalement côte à côte, d'une part un siège de fixation (5) dudit tube rigide (7), dont l'extrémité proximale communique de façon étanche avec ladite lumière de retour (3) et d'autre part ladite lumière de prélèvement (4), l'extrémité distale (2a) dudit embout formant ainsi une brusque augmentation de section de ladite aiguille située dans le prolongement de ladite lumière de prélèvement (4).

2. Aiguille selon la revendication 1, dans laquelle la section de la partie distale (2a) dudit embout (2) est sensiblement constante, de son extrémité distale jusqu'à une certaine distance de ladite extrémité d'admission (8) en direction proximale.

3. Aiguille selon l'une des revendications précédentes, dans laquelle le diamètre dudit embout (2) à proximité de son extrémité distale (2a) est inférieure au double du diamètre dudit tube rigide (7), alors que les sections respectives correspondantes des conduits (7, 4) desdites lumières dans cette partie distale (2a) dudit embout (2) sont sensiblement égales.

4. Aiguille selon l'une des revendications précédentes, dans laquelle plus de 80% du diamètre dudit tube rigide (7) est situé à l'intérieur du diamètre de la partie d'extrémité distale (2a) dudit embout (2).

5. Aiguille selon l'une des revendications précédentes, dans laquelle la cloison (9) de la portion distale (2a) dudit embout (2) séparant ledit siège (5) dudit tube rigide (7) de ladite lumière de prélèvement (4) est de section courbe concentrique à l'axe longitudinal dudit tube rigide (7), la section de ladite lumière de prélèvement (4) étant ainsi essentiellement délimitée par deux courbures, une courbure interne, concentrique à l'axe dudit tube rigide (7) et une courbure externe concentrique à l'axe dudit embout (2).

6. Aiguille selon l'une des revendications précédentes, dans laquelle l'extrémité d'admission (8) qui s'ouvre latéralement dans la paroi dudit embout (2) comporte une pluralité d'ouvertures.

7. Aiguille selon l'une des revendications précédentes, dans lequel ledit tube rigide (7) est en acier inoxydable.

8. Aiguille selon l'une des revendications précédentes, dans lequel ledit embout (2) est en matière plastique.

9. Procédé de fabrication d'une aiguille à deux lumières selon la revendication 8, selon lequel ledit embout (2) est surmoulé sur ledit tube rigide (7).

10. Procédé de fabrication selon la revendication 10, selon lequel on forme au moins une ouverture latérale (7b) dans la partie proximale de la paroi dudit tube rigide (7) pour permettre à ladite matière plastique de l'embout (2) de pénétrer dans cet ouverture latérale (7b) pour solidariser l'embout (2) audit tube rigide (7).
